# EUROPEAN PATENT APPLICATION

(11) **EP 2 765 132 A1**
(43) Date of publication of application: **13.08.2014**
(21) Application number: 13154695.4
(22) Date of filing: 08.02.2013
(51) Int. Cl.: C07D 417/14, C07D 487/22, C09B 57/10, H01L 51/00

(54) **Dye compounds, and their use in dye-sensitized solar cells**

(71) Applicant: Solvay SA, 1120 Bruxelles (BE)
(72) Inventor: The designation of the inventor has not yet been filed
(74) Representative: Mross, Stefan P.M.

(57) **Abstract**

Disclosed are compounds of formula D-B-A-ANC (wherein D is an electron donor group, B is a chromophore group, A is an electron acceptor group, and ANC is an anchoring part comprising at least one anchoring group, and dye-sensitized solar cells (DSSC) incorporating the same as a dye-sensitizer.

## Description

### TECHNICAL FIELD

The present invention relates to novel dye compounds, methods of making the same, and their use as dyes in photoelectric conversion devices, especially in dye-sensitized solar cells (DSSC).

### BACKGROUND OF THE INVENTION

Conventional solar cells convert light into electricity by exploiting the photovoltaic effect that exists at semiconductor junctions. In other words, the commercial solar cells absorb energy from visible light and convert excited charge carriers thereof to electric energy. At present, the main commercial solar cells are silicon-based solar cells. For the silicon-based solar cell, there are shortcomings in that high energy costs for material processing is required and many problems to be addressed such as environmental burdens and cost and material supply limitations are involved. For an amorphous silicon solar cell, there are also shortcomings in that energy conversion efficiency decreases when used for a long time, due to deterioration in a short period.

Recently, attempts have been undertaken to develop low-cost organic solar cells. In particular, developments have targeted dye-sensitized solar cells (DSSC) which are based on a semiconductor electrode sensitized by dye-sensitizer, a counterelectrode and an electrolyte. The dye-sensitizer absorbs incoming light to produce excited electrons.

Meanwhile, organic dye-sensitizers of which use is suitable for DSSC have been developed. Usually, the organic dye-sensitizers comprises three parts, that are, electron donor group (D), electron acceptor group (A) and π-electron bridge group (B). One particular class of the organic dye-sensitizers is the compound wherein the π-electron bridge group comprises a chromophore group, such as porphyrin. This class is a subject of interest for organic type dye-sensitizer in DSSC due to the intrinsic useful optical, structural, and electrochemical properties. For example, the United States Patent Application Publication No. US 2010/125136 Al discloses photosensitizer dyes having certain porphyrin structure for dye-sensitized solar cells.

### DESCRIPTION OF THE INVENTION

The purpose of the present invention is to provide novel dye compounds particularly having advantageous performance(s) when used in photoelectric conversion devices, especially in particular in dye-sensitized solar cells (DSSC).

The present invention therefore relates to compounds of formula (I) :

D-B-A-ANC (I)

wherein D is an electron donor group, B is a chromophore group, A is an electron acceptor group, and ANC is an anchoring part comprising at least one anchoring group.

The compounds of D-B-A-ANC type according to the present invention are constructed through the linkage of effective electron acceptor group (A) and electron donor group (D) substituted on chromophore π system, inducing an intramolecular charge transfer that gives rise to wide-range of wavelength absorption as well as excellent directionality in excited state. Also, the compounds of the present invention enables photoelectric conversion device, particularly DSSC, with very high efficiency.

Indeed, it has been surprisingly found that the organic dye compounds at least comprising chromophore structure substituted with electron acceptor group and electron donor group of the D-B-A-ANC type organic dye provide excellent characteristics in DSSC, especially concerning their efficiency. Without wishing to be bound by any particular theory, it can be assumed that certain linkage between different photo- and electro-active π-systems which are separately tunable results in perturbations of the absorption and electrochemical properties of the resulting compounds, creating a new class of organic dye compounds with unexpectedly promoted electronic coupling for realizing efficient DSSC.

In the present invention, "alkyl groups" is understood to denote in particular a straight chain, branched chain, or cyclic hydrocarbon groups usually having from 1 to 20 carbon atoms. Examples of alkyl groups include methyl, ethyl, propyl, isopropyl, butyl, isobutyl, sec-butyl, tert-butyl, pentyl, neopentyl, hexyl, cyclopropyl, cyclobutyl, cyclopentyl, and cyclohexyl.

In the present invention, "alkoxy groups" is understood to denote in particular a straight chain, branched chain, or cyclic hydrocarbon groups usually having from 1 to 20 carbon atoms singularly bonded to oxygen (Alk-O-).

In the present invention, "alkylthio groups" is understood to denote in particular a straight chain, branched chain, or cyclic hydrocarbon groups usually having from 1 to 20 carbon atoms singularly bonded to sulfur atom (Alk-S-).

In the present invention, "aryl groups" is understood to denote in particular any functional group or substituent derived from an aromatic ring. In particular, the aryl groups can have 6 to 20 carbon atoms preferably 6 to 12 carbon atoms, in which some or all of the hydrogen atoms of the aryl group may or may not be substituted with other groups, especially alkyl groups, alkoxy groups, aryl groups, or hydroxyl groups. The aryl groups are preferably optionally substituted phenyl groups, naphthyl groups, anthryl group and phenanthryl group. In the present invention, the aryl groups substituted by alkoxy groups may alternatively be named as "alkoxyaryl groups."

In the present invention, "aryloxy groups" is understood to denote in particular the aryl group as defined above singularly bonded to oxygen (Ar-O-).

In the present invention, "heterocycles" is understood to denote in particular a cyclic compound which has at least one heteroatom as a member of its one or more rings. Frequent heteroatoms within the ring include sulfur, oxygen and nitrogen. The heterocycles can be either saturated or unsaturated, and may be 3-membered, 4-membered, 5-membered, 6-membered or 7-membered ring. The heterocycles can be further fused with other one or more ring systems. Examples of the heterocycles include pyrrolidines, oxolanes, thiolanes, pyrroles, furans, thiophenes, piperidines, oxanes, thianes, pyridines, pyrans, thiopyrans, azepanes, oxepanes, thiepanes, azepines, oxepines, thiepines, triazoles, furazans, oxadiazoles, thiadiazoles, dithiazoles, piperazines, morpholines, thiomorpholines, dioxanes, dithianes, diazines, oxazines, thiazines, dioxines, dithiines, and their derivatives. The heterocycles can further be substituted by other groups, such as alkyl groups, alkoxy groups, alkylthio groups, aryl groups or aryloxy groups as defined above.

In the present invention, "amino groups" is understood to denote in particular aliphatic amines or aromatic amines and encompass compounds comprising one or multiple amino groups. The amino groups can be primary amines, secondary amines and tertiary amines, wherein one or more hydrogen atoms may or may not be independently substituted with other groups, such as optionally substituted alkyl groups, optionally substituted alkoxy groups, optionally substituted alkylthio group, optionally substituted aryl group, optionally substituted aryloxy groups, or optionally substituted alkoxyaryl groups.

In the present invention, "halogenated" is understood to denote in particular at least one of the hydrogen atoms of the following chemical group has been replaced by a halogen atom, preferably selected from fluorine and chlorine, more preferably fluorine. If all of the hydrogen atoms have been replaced by halogen atoms, the halogenated chemical group is perhalogenated. For instance, "halogenated alkyl groups" include (per)fluorinated alkyl groups such as (per)fluorinated methyl, ethyl, propyl, isopropyl, butyl, isobutyl, *sec*-butyl or *tert*-butyl ; and for instance -CF₃, -C₂F₅, heptafluoroisopropyl (-CF(CF₃)₂), hexafluoroisopropyl (-CH(CF₃)₂) or -CF₂(CF₂)₄CF₃. Non-limiting example of "halogenated aryl groups" include -C₆F₅. Those substituted heterocycles may alternatively be named as "heterocyclic alkyl groups" when alkyl groups is substituted, "heterocyclic alkoxy groups" when alkoxy groups is substituted, "heterocyclic alkylthio groups" when alkylthio groups is substituted, "heterocyclic aryl groups" when aryl groups is substituted, "heterocyclic alkoxyaryl groups" when alkoxyaryl groups is substituted, or "heterocyclic aryloxy groups" when aryloxy groups is substituted

In the present invention, the chromophore group preferably comprises at least one non-metal or metal complex chromophore. In particular, the chromophore group comprises at least one macrocylic non-metal or metal complex chromophore. More specifically, the chromophore group comprises at least one macrocyclic non-metal or metal complex chromophore comprising porphyrin, benzoporphyrin, phthalocyanine, naphthalocyanine or azulenocyanine structure. The porphyrin, benzoporphyrin, phthalocyanine, naphthalocyanine or azulenocyanine structure can be substituted by at least one substituents, each independently selected from, but not limited to, the group consisting of halogens, cyano, alkyl groups, halogenated alkyl groups, alkoxy groups, halogenated alkoxy groups, alkylthio groups, halogenated alkylthio groups, aryl groups, halogenated aryl groups, aryloxy groups, halogenated aryloxy groups, alkoxyaryl groups, halogenated alkoxyaryl groups, heterocycles, halogenated heterocycles, heterocyclic alkyl groups, halogenated heterocyclic alkyl groups, heterocyclic alkoxy groups, halogenated heterocyclic alkoxy groups, heterocyclic alkylthio groups, halogenated heterocyclic alkylthio groups, heterocyclic aryl groups, halogenated heterocyclic aryl groups, heterocyclic aryloxy groups, halogenated heterocyclic aryloxy groups, heterocyclic alkoxyaryl groups, halogenated heterocyclic alkoxyaryl groups, amino groups, and halogenated amino groups. The most preferred chromophore in the present invention is a porphyrin. More preferably, the porphyrin is selected from the following structures : 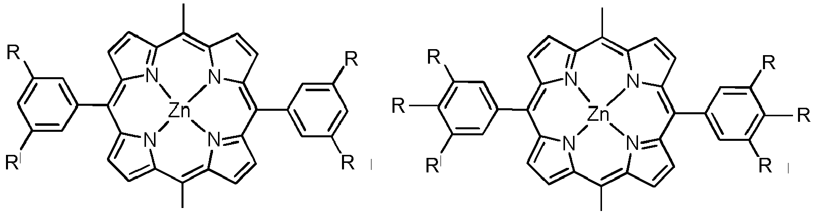 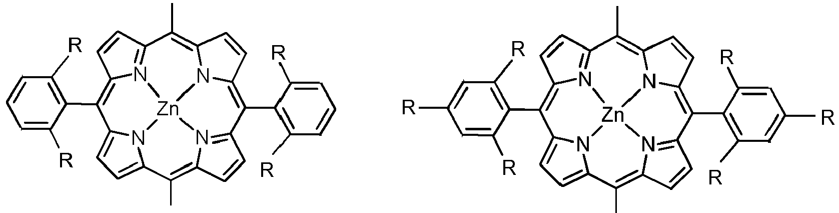 wherein R is independently selected from the group consisting of hydrogen, -CN, optionally branched alkyl, alkoxy and alkylthio groups and optionally substituted aryl, aryloxy and alkoxyaryl groups, particularly from optionally branched C1-C18 alkyl, alkoxy and alkylthio groups and C5-C12 aryl groups substituted by C8-C12 alkoxy groups, more particularly t-butyl, -OC₈H₁₇ or -OC₁₂H₂₅.

In the present invention, the electron acceptor group A has highly electron deficient nature, which makes itself be able to function as strongly electron withdrawing group. This property of the electron acceptor group enables panchromatic absorption, leading to higher efficiency. In the present invention, the electron acceptor group A of the compound having formula (I) preferably comprises at least one group selected from the following structures or any combination thereof, but not limited thereto : 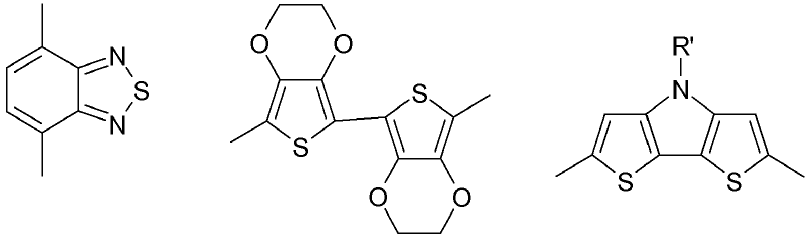 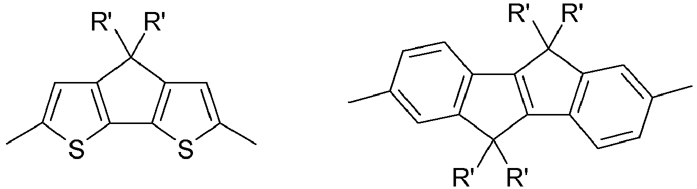 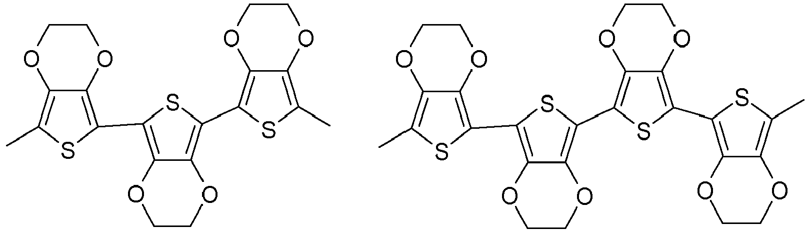 wherein R' are independently selected from the group consisting of optionally branched alkyl, alkoxy and alkylthio groups and optionally substituted aryl, aryloxy and alkoxyaryl groups, particularly from optionally branched C1-C18 alkyl, alkoxy and alkylthio groups and optionally substituted C5-C12 aryl groups, particularly from optionally branched C6-C12 alkyl groups and optionally substituted C6 aryl groups, more particularly from linear C6-C12 alkyl groups and phenyl group.

More preferably, the electron acceptor group comprises benzothiadiazole structure. In case the electron acceptor group comprises benzothiadiazole, the portion of A-ANC of the formula (I) can be selected from the following examples, but the present invention is not limited thereto.

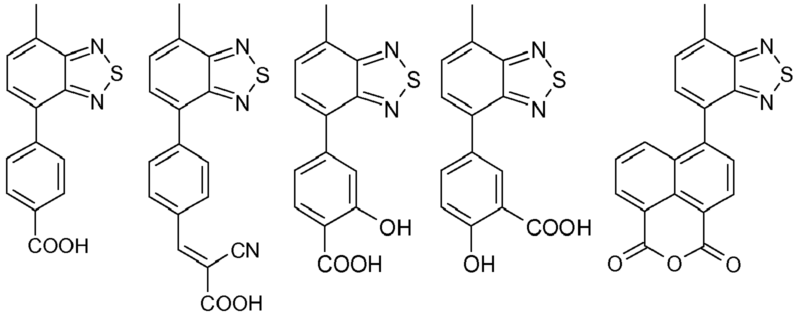

In this particular embodiment where the electron acceptor group according to the present invention comprises benzothiadiazole, the at least one anchoring group, such as -COOH, salicylic acid or acrylic acid anchoring group, may be directly attached to benzothiadiazole acceptor group. The particular examples of A-ANC structure according to this construction are as follows, but the present invention is not limited thereto.

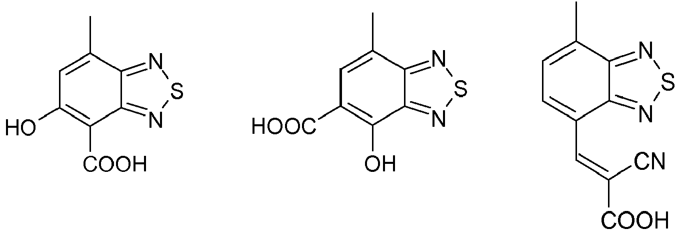

In the present invention, the electron donor group D in the compound having formula (I) preferably includes optionally substituted amine group. More preferable electron donor group D in the present invention includes amino group substituted by two or three aryl groups, such as phenyl, the aryl groups optionally being independently substituted with further optionally substituted aryl groups or alkoxyaryl groups. Still more preferably, the electron donor group comprises at least one group selected from the following structures, but the present invention is not limited thereto : 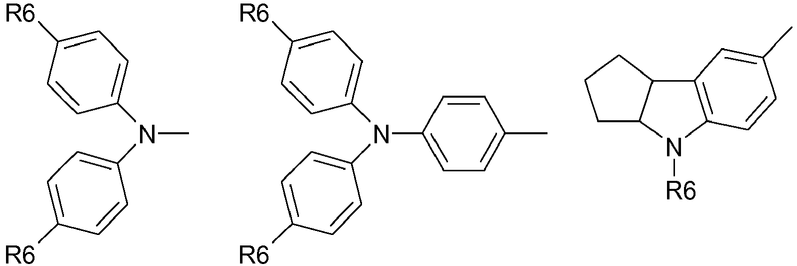 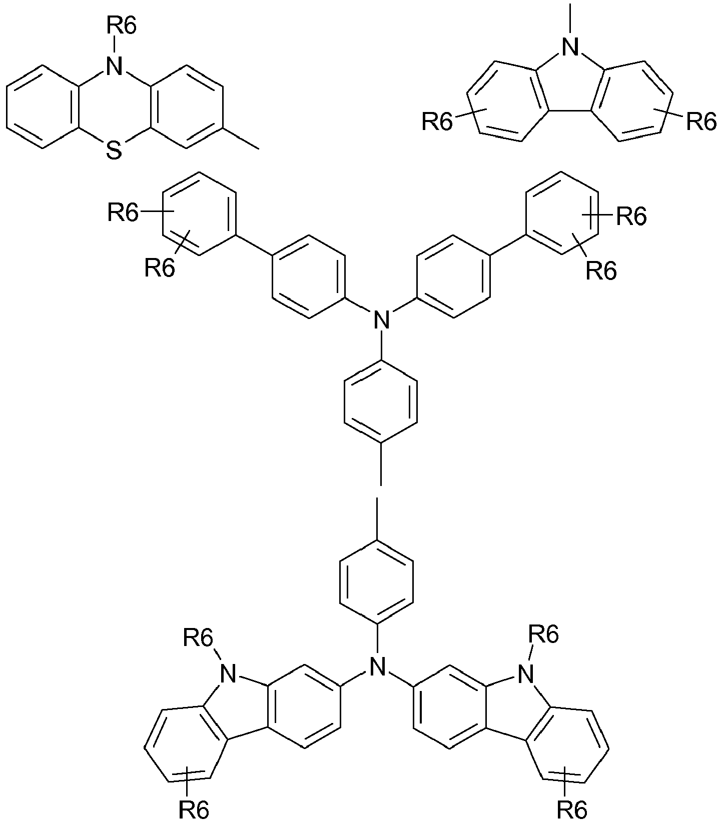 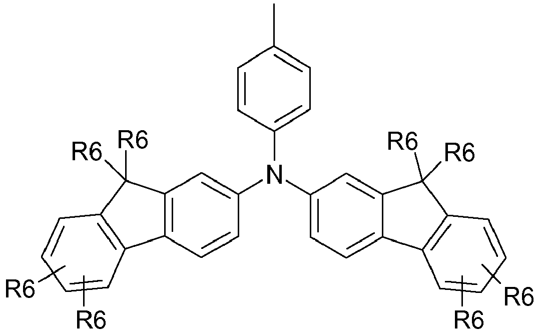 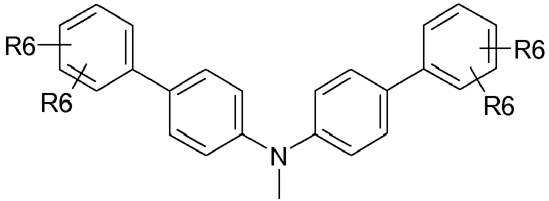 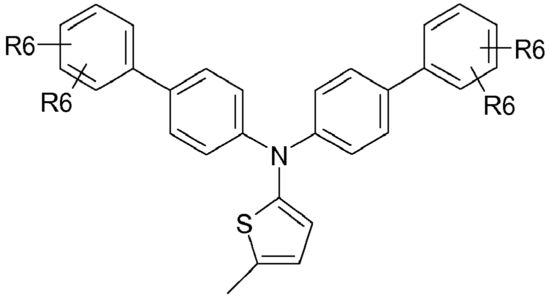 wherein R6 are independently selected from the group consisting of hydrogen, -CN, optionally branched C1-C18 alkyl, alkoxy and alkyothio groups and optionally substituted C6-C12 aryl, aryloxy and alkoxyaryl groups, preferably from optionally branched C1-C12 alkyl, alkoxy or alkylthio groups.

In a particular embodiment, the electron donor group (D) and the chromophore group (B), and/or the electron acceptor group (A) and the chromophore group (B) are linked through an optional electron bridge group. Without wishing to be bound by any theory, the optional electron bridge group at least has a function to electronically conjugate two or more π-systems, that is, for instance, conjugate D to B and/or B to A of the formula (I) according to the present invention. Said optional electron bridge group may be independently selected from aromatic ring system, alkene group, polyene system, alkyne group, polyyne system or heteroatom-containing variants of such systems wherein one or more carbon atoms and/or one or more C=C double bonds are replaced by a heteroatom. Preferably, the optional electron bridge group can be selected from alkene group, polyene system, alkyne group or polyyne system. The optional electron bridge group may be selected from heteroatom-containing derivatives of the aromatic ring system, such as thiophene derivatives, which can be condensed to the aromatic structure represented in formula (I).

In the present invention, at least one anchoring group is contained in the compound having formula (I).

In the present invention, "anchoring part" is understood to denote in particular a part which comprises at least one spacer group, which is usually an aromatic group, and at least one anchoring group.

In a particular embodiment of the present invention, the anchoring part ANC comprises at least one aromatic group and at least one anchoring group. The examples of the aromatic group in this embodiment are phenyl or naphthyl group, or heterocycles optionally fused with other ring system. Preferred aromatic group of the anchoring part is phenyl group. The representative examples of the anchoring part are as follows, but the present invention is not limited thereto.

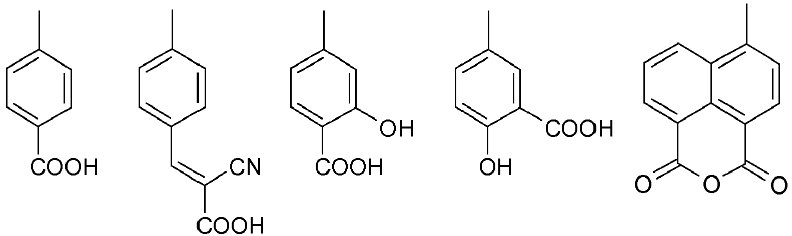

In the present invention, "anchoring group" is understood to denote in particular groups that will allow attachment of the dye-sensitizers onto a semiconductor material, such as TiO₂. The term "anchoring" is understood to denote in particular a chemical or physicochemical interaction between the dye-sensitizer and the semiconductor material, e.g. by means of a covalent bond, an ionic bond or a hydrogen bond. Suitable anchoring groups are for instance selected from, but not limited to, the group consisting of -COOH, -PO₃H₂, - PO₄H₂, -SO₃H, -CONHOH, acetylacetonate, acrylic acid derivatives, malonic acid derivative, rhodanine-3-acetic acid, propionic acid, salicylic acid, formic anhydride, deprotonated forms of the aforementioned, salts of said deprotonated forms, and chelating groups with π-conducting character, more preferably - COOH or the salts of deprotonated -COOH. The acrylic acid derivatives may for instance be selected from groups of formula -CH=C(R5)-COOH where R5 is selected from H, CN, COOH and alkyl groups substituted by at least one halogen atom, preferably from H, CN, COOH and CF3. The malonic acid derivatives suitable as anchoring groups may for example be selected from groups of formula -CR4=C(COOH)₂ where R4 is selected from H and optionally halogenated alkyl groups, especially from H and optionally fluorinated alkyl groups. Especially preferable salts of -COOH are for instance ammonium salts, or alkali metal salts, more preferably -COOTBA (wherein, TBA indicates tetrabutylammonium).

In the present invention, the electron acceptor group (A) and the anchoring part (ANC) can be linked through an optional electron bridge group. Said optional electron bridge group may be independently selected from alkene group, polyene system, alkyne group, polyyne system or heteroatom-containing variants of such systems wherein one or more carbon atoms and/or one or more C=C double bonds are replaced by a heteroatom.

The most preferred embodiment of the present invention comprises the partial structure of formula (I) which is D-B-A, wherein D is optionally substituted diaryl or triaryl amine group, B is a porphyrin group and A is a benzothiadiazole group. Thus, the present invention also concerns the compound comprising D-B-A part, wherein optionally substituted diaryl or triaryl amine group, B is a porphyrin group and A is a benzothiadiazole group.

In the present invention, the exemplary compounds according to the present invention can be selected from the following structures : 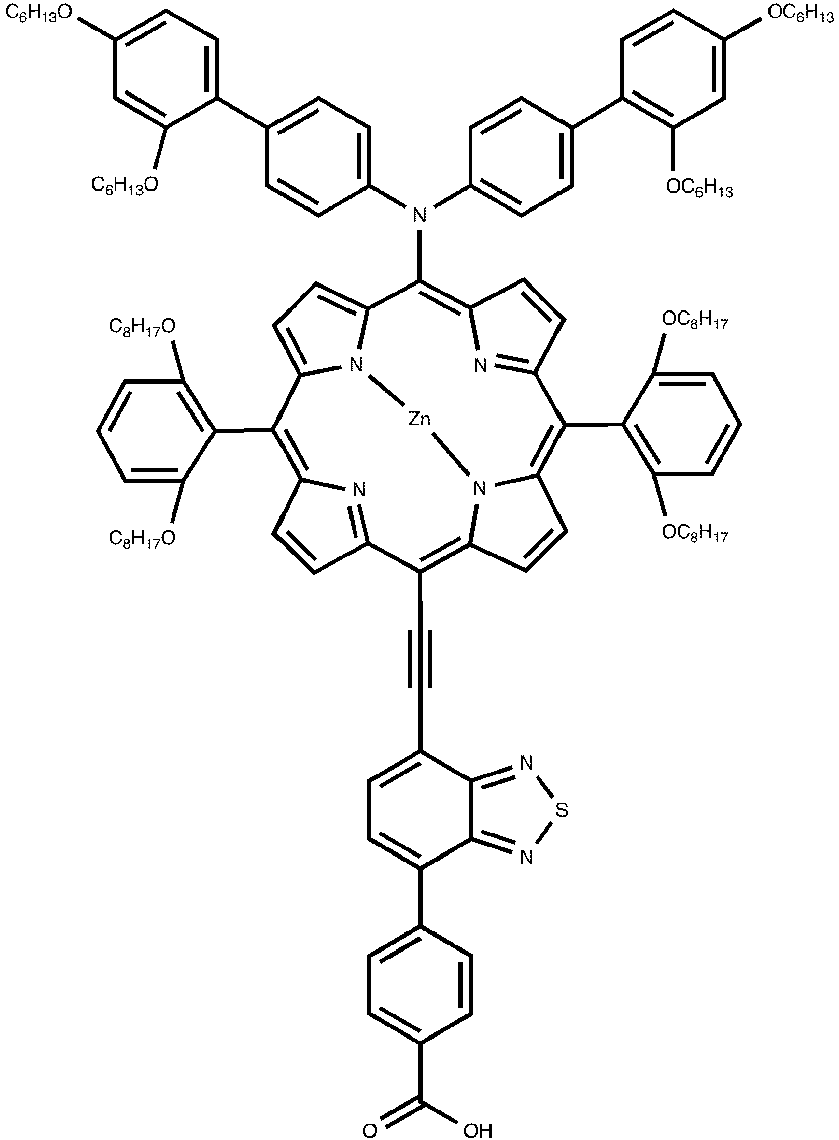 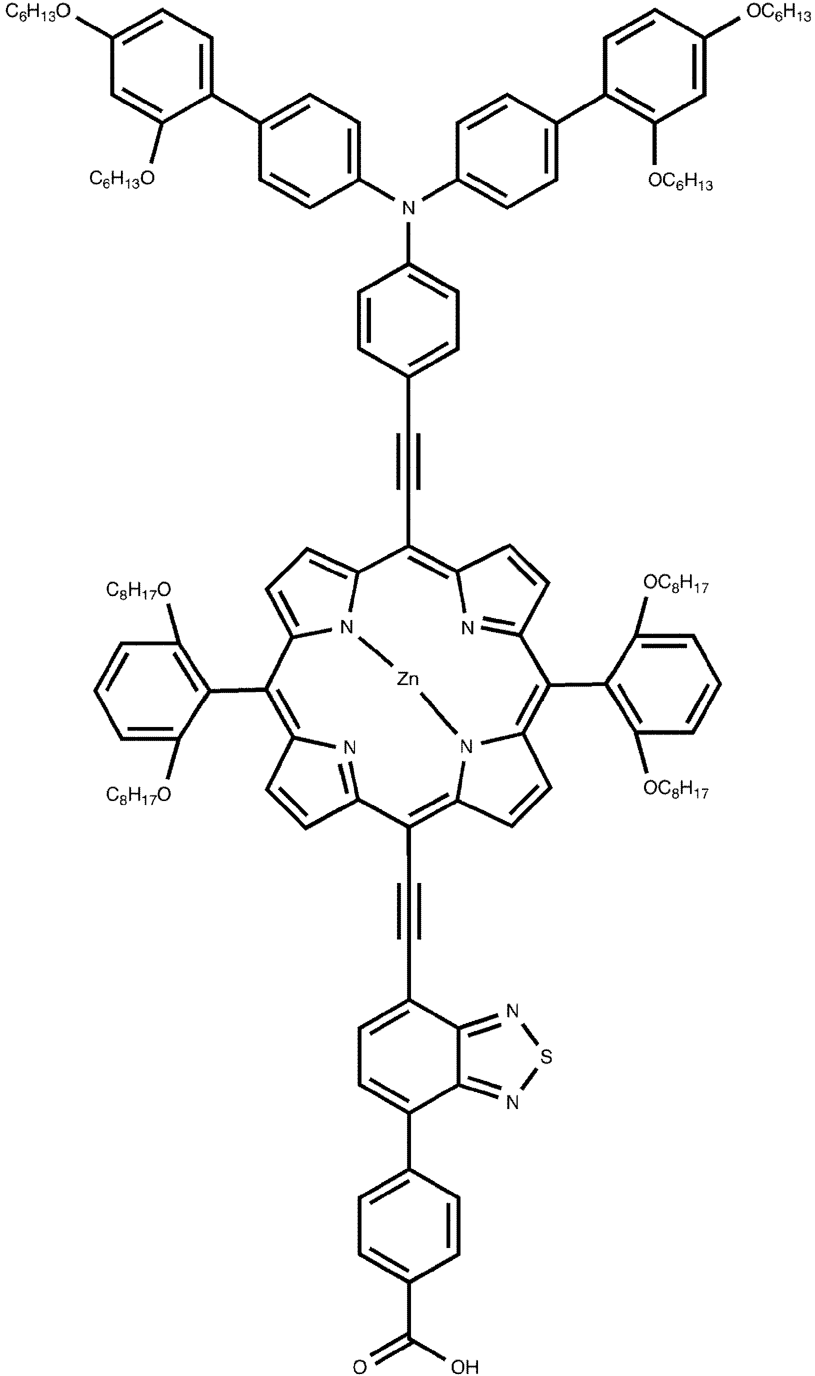 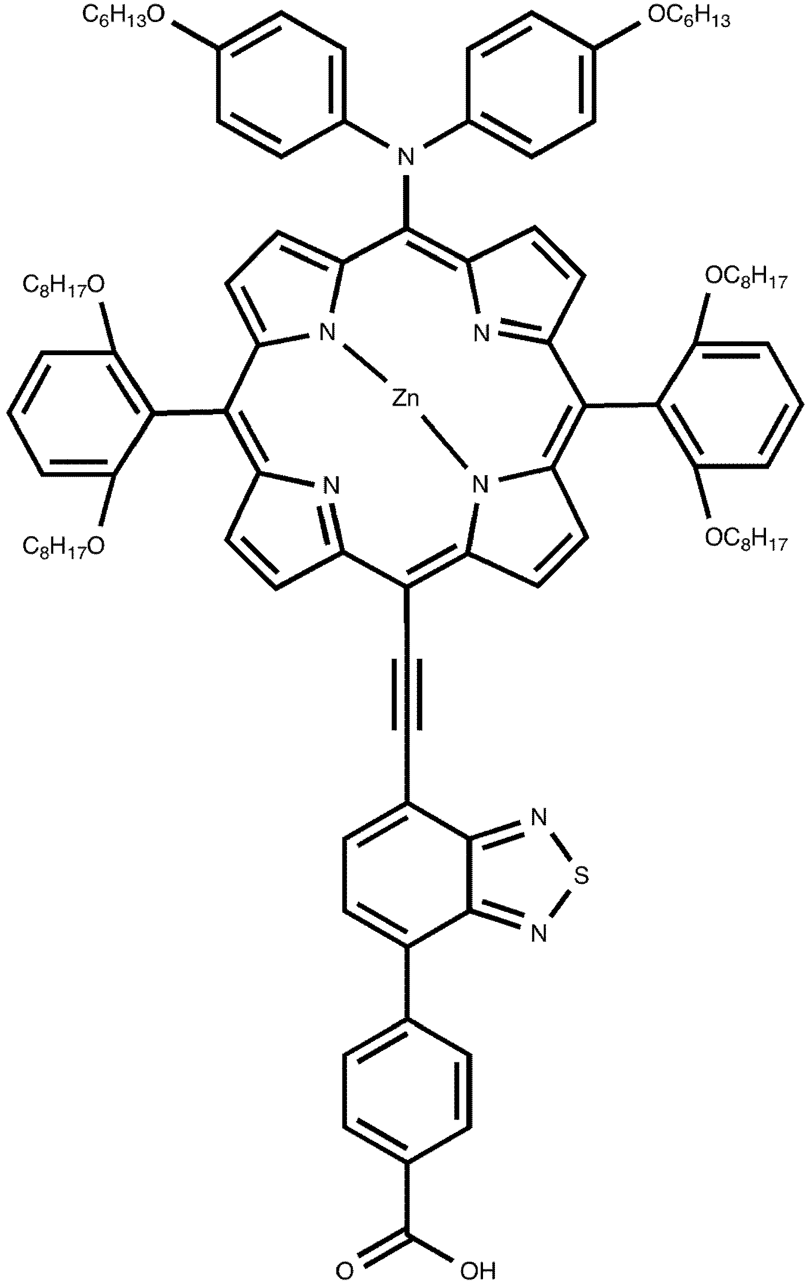 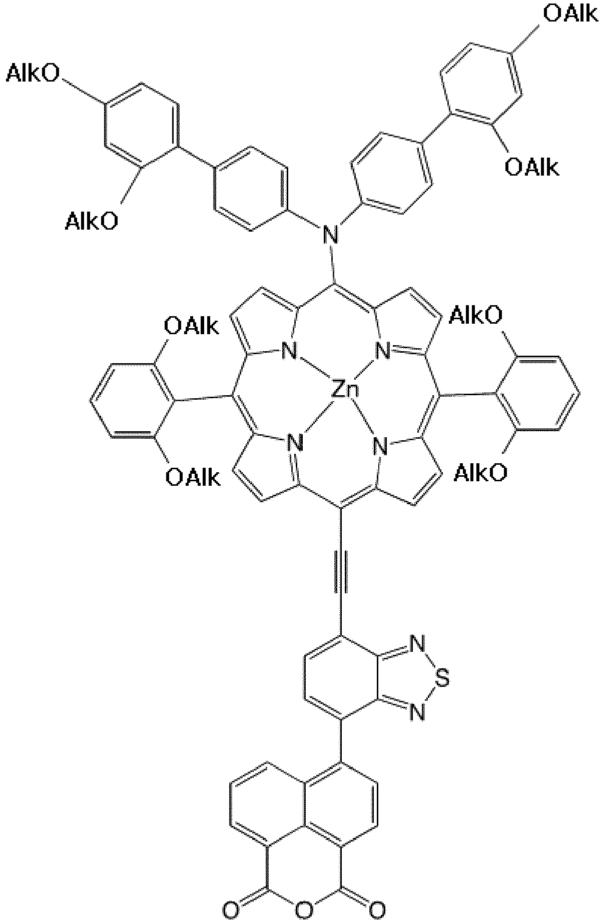 wherein Alk is alkyl group.

The compounds of the present invention can be electronically neutral or neutralized with counter-ion resulting in a salt form. Examples of these salts include ammonium salts and alkali metal salts of the compounds of the present invention. Therefore, the present invention also relates to a salt of the compound according to the present invention.

The compounds of the present invention described herein can be a dye-sensitizer which may be suitable for use in photoelectric conversion devices, especially in dye-sensitized solar cells (DSSC). The present invention therefore also relates to the use of a compound of the present invention or its salt in photoelectric conversion devices, especially in DSSC.

The DSSC offers the prospect of a low cost and versatile technology for large scale production of solar cells. The dye-sensitized solar cell (DSSC) is formed by a combination of organic and inorganic components that could be produced at a low cost. The dye-sensitized solar cells have advantages over silicon-based solar cells in terms of simplified processing steps, low fabrication cost, and transparency. The dye-sensitized solar cells can be fabricated from flexible substrates to function as cells of mobility and portability. The dye-sensitized solar cells have also the advantage to be lightweight.

One of the objectives that dye-sensitized solar cells are facing over the silicon-based solar cells is to increase relatively lower energy (photoelectric) conversion efficiency. In order to improve the energy conversion efficiency, panchromatic absorption spectra would be of interest.

One of the basic elements of a DSSC is generally a semiconductor material sensitized with dye-sensitizers to form the core of a DSSC. The semiconductor material is preferably selected from the group of titanium salts, tin salts and zinc salts, more preferably the semiconductor material is selected from the group of TiO₂, SnO₂, ZnO and TiOF₂. Those semiconductor material can be in form of nanoparticles. TiO₂ (titanium dioxide) is a preferred semiconductor material since its surface is highly resistant to the continued electron transfer. TiOF₂ (titanyl oxyfluoride, titanium oxyfluoride or titanium fluoride oxide) can also be envisaged as a suitable semiconductor. TiOF₂ is especially suitable when combined with fluorinated dyes. The materials can be used as the sole semiconductor in the DSSC semiconductor layer or can be combined in mixture with any other suitable semiconductor layer material such as gallium oxide etc. The dye-sensitizers anchored to the semiconductor material's surface are used to harvest a great portion of the solar light.

The dye-sensitizers are sensible to the visible light. The light excites electrons from highest occupied molecular orbitals (HOMO) to lowest unoccupied molecular orbitals (LUMO), which is rapidly injected to the semiconductor particles (usually TiO₂). The particulate semiconductor functions as the transporter of light induced electrons towards the external contact, a transparent conductor that lies at the basis of the semiconductor (e.g. TiO₂) film.

Construction of a DSSC is well known to the person skilled in the art. Generally, the DSSC comprises an anode, a cathode, and an electrolyte. The anode and cathode are arranged in a sandwich-like configuration, and the electrolyte is inserted between the two electrodes.

The material for the cathode is not limited as long as the cathode is formed from a material having conductivity. For a non-limiting example, a substrate comprising electrically conductive transparent glass which contains a small amount of platinum or conductive carbon adhering to the surface can be suitably used. As the electrically conductive transparent glass, a glass made of tin oxide or indium-tin oxide (ITO) can be used.

The anode has a substrate made of electrically conductive transparent glass and a semiconducting layer comprising a semiconductor material and the dye-sensitizer of the present invention adsorbed thereto. As an example of the electrically conductive transparent glass for the anode, a glass comprising the materials described above for the cathode can be used, but not limited to them.

The dye-sensitizer of the present invention is caused to be adsorbed on the semiconductor material. The dye is adsorbed by causing the anode comprising substrate of electrically conductive transparent glass and a semiconducting layer formed on the surface to come in contact with a dye solution containing the dye-sensitizer of the present invention and a solvent. The association of the dye-sensitizer and the semiconductor can be maintained through chemical bond or electrostatic interaction achieved between the anchoring group of the dye compound and the semiconductor material. Other methods than the adsorption for achieving association of the dye and the semiconductor are known to the person skilled in the art.

As the electrolyte, a liquid electrolyte, a solid electrolyte, or a solution containing the electrolyte can be used. The electrolyte is preferably a redox electrolyte, containing a substance forming a redox system, for example a solution containing iodine and imidazolium salt of iodide, which forms a redox system of I₃⁻ + 2 e⁻ ↔ 3 I⁻ + I₂. As the suitable solvent for the solution, an electrochemically inert substance, such as acetonitrile or propionitrile can be used. In one specific embodiment according to the present invention, the electrolyte may comprise a complex of a first row transition metal, preferably cobalt complex as a redox mediator. In a further embodiment of the present invention, the cobalt complex has the formula (A) :

[L¹ₐCoL²_{b}](X_{c}) (A)

wherein Co is cobalt ; a and b are independently integer from 0 to 6, c is integer from 0 to 3 ;
each X is independently a counter-anion, preferably selected from borates or phosphates ;
each L¹ is independently selected from the group consisting of unsubstituted or substituted bipyridine ligands and unsubstituted or substituted terpyridine ligands ; and
each L² is independently a co-ligand, preferably selected from the group consisting of Cl-, Br-, I-, CN-, NCO-, NCS-, NCSe-, amino groups, phosphate groups, ClO₄⁻, PF₆⁻, borates such as [BF₄]⁻, tetracyanoborate ([B(CN)₄]⁻), [B(Ph)₄]⁻, [B(C₆F₅)₄]⁻, bis(trifluoromethanesulfonyl)imide (TFSI⁻), bis(fluorosulfonyl)imide (FSI⁻), and any combination thereof. Another examples of such complex include, but not limited to, cobalt polypyridine complexes disclosed in WO 2003/038508, cobalt trisbipyridyl complex in [Feldt, S. et al., J. Am. Chem. Soc. 2010, 132, 16714-16724] and the cobalt complexes containing at least one mono-, bi- or tridentate ligand comprising a substituted or unsubstituted ring system comprising a five-membered or six-membered ring comprising at least one heteroatom, preferably nitrogen atom and at least one double bond.

The DSSC can be formed, for example, by filling with the electrolytic solution between the two electrodes face to face. The two electrodes can be thus disposed with a desired distance between them by securing them by sandwiching a spacer between them. Nonetheless, other methods of manufacturing the DSSC would be well understood by a person skilled in the art.

The present invention also provides methods for manufacturing the compound according to the present invention, which comprises reacting the electron donor group D or its precursor and the π-electron bridge group B or its precursor, and further reacting the resultant with the electron acceptor group A or its precursor. The elucidation for the methods for manufacturing the compound would be furthered in the example below.

The compound of the present invention may be further isolated, for instance, by column chromatography, preferably by high pressure liquid chromatography (HPLC).

While preferred embodiments of this invention have been shown and described, modifications thereof can be made by one skilled in the art without departing from the spirit or teaching of this invention. The embodiments described herein are exemplary only and are not limiting. Many variations and modifications of systems and methods are possible and are within the scope of the invention. Accordingly, the scope of protection is not limited to the embodiments described herein, but is only limited by the claims that follow, the scope of which shall include all equivalents of the subject matter of the claims.

### SHORT DESCRIPTION OF THE FIGURES

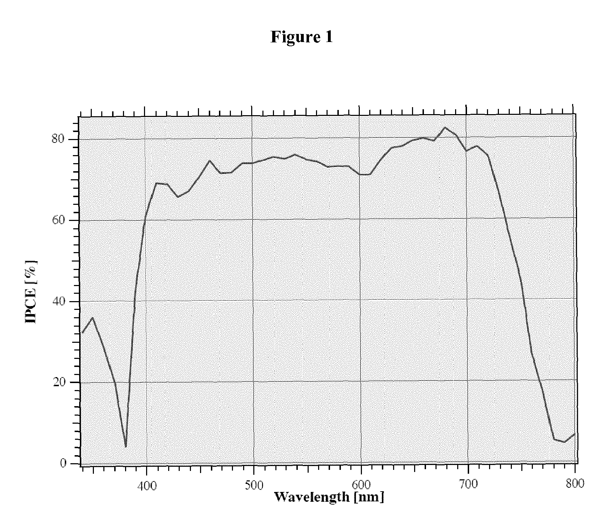

Figure 1 shows Incident Photon to Current Conversion Efficiency of the compound of formula (I-1) according to Example 1.

### Examples

### Example 1 : Synthesis of dye compound (I-1)

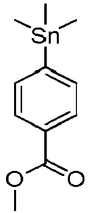

Methyl 4-iodobenzoate (2.62 g, 10 mmol), PdCl₂(PPh₃)₂ (0.1 mmol, 70.1 mg, 1 mol %) was evacuated and purged with N₂ before adding 1,4-dioxane (100 mL) and hexamethyldistannane (5.00 g, 15 mmol), and the resulting solution stirred at 60°C for 15 hours. The solvents were evaporated and the residue dried on high vaccum prior to purification via column chromatography (silica, hexane) to afford the desired product (3.00 g, 99 %) as a colourless oil.

¹H NMR (400 MHz CDCl₃) *δ* 7.97 (d, J= 8.1 Hz, 2H), 7.58 (d with Sn satellites, J= 8.1 Hz, 2H), 3.91 (s, 3H), 0.32 (s with Sn satellites, 9H) ; APCI-FTMS (M⁺), calc. 301.02505, obs. 301.02445.

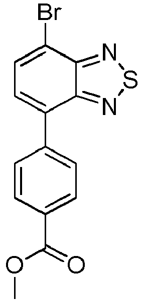

Methyl 4-(trimethylstannyl)benzoate (210 mg, 0.702 mmol, 1.25 equiv), 4,7-bromo-2,1,3-benzothiadiazole (165 mg, 0.562 mmol), CsF (212 mg, 1.404 mmol, 2 equiv), PdCl₂ (5 mg, 0.0281 mmol, 5mol %), *t*-Bu₃P (56 µL, 1M in toluene, 0.0562 mmol, 10mol %), CuI (5 mg, 0.0281 mmol, 5mol %) and DMF (2.4 mL) were stirred at 45°C for 1 hour, upon which no more stannane starting material was detected by TLC (silica, EtOAc/hexane, 1:4). The reaction mixture was diluted with EtOAc (50 mL) and washed with water (5 × 50 mL), dried (Na₂SO₄) and evaporated prior to purification using column chromatography (silica, EtOAc/hexane, 1:4) to afford the desired product as a yellow solid (73 mg, 37 %).

¹H NMR (400 MHz, CDCl₃) *δ* 8.20 (d, J= 8.4 Hz, 2H), 7.99 (d, J= 8.4 Hz, 2H), 7.96 (d, J = 7.6 Hz, 1H), 7.64 (d, J = 7.6 Hz, 1H), 3.97 (s, 3H) ; APCI-FTMS (M⁺), calc. 347.95626, obs. 347.95618.

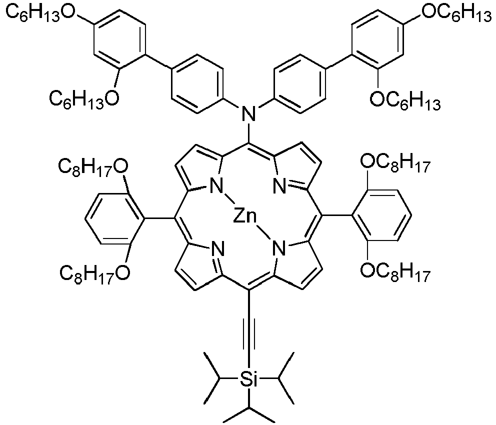

To a solution of **Br-ZnP(OC8)-TIPSA¹** (500 mg, 0.3852 mmol), Pd(OAc)₂ (8.7 mg, 0.0385 mmol, 10mol %) and DPEPhos (41.5 mg, 0.077 mmol, 20 mol %) in THF (30 mL) was added a solution of bis(4-(2,4-dihexyloxyphenyl)phenyl)amine² (333 mg, 0.4622 mmol, 1.2 equiv) and NaH (60 % in mineral oil, washed with hexane, 46.2 mg, 1.156 mmol, 2.5 equiv) in THF (4 mL), washing with THF (4 mL) to ensure complete transferral of the amide solution. The resulting reaction mixture was stirred at reflux for 15 hours before cooling and partitioning between water and DCM. The organics were dried (Na₂SO₄), evaporated and separated through column chromatography (silica, DCM/hexane, 1:2) to afford the desired product (384 mg, 54 mg) as a green solid.

¹H NMR (400 MHz, CDCl₃) *δ* 9.59 (d, *J*= 4.4 Hz, 2H), 9.11 (d, *J*= 4.4 Hz, 2H), 8.80 (d, *J*= 4.8 Hz, 2H), 8.65 (d, *J*= 4.8 Hz, 2H), 8.46 (t, *J*= 8.4 Hz, 2H), 7.20 (d, *J*= 8.6 Hz, 4H), 7.15-7.07 (m, 6H), 6.96 (d, *J*= 8.4 Hz, 4H), 6.48 (d, *J* = 2.2 Hz, 2H), 6.44 (d, *J* = 2.2 Hz, 1H), 6.42, (d. *J* = 2.2 Hz, 1H), 3.91 (t, *J*= 6.6 Hz, 4H), 3.89 (t, *J*= 6.6 Hz, 4H), 3.82 (t, *J*= 6.6 Hz., 8H), 1.80-1.65 (m, 8H), 1.48-1.33 (m, 8H), 1.32-1.20 (m, 16H), 1.01-0.80 (m, 28H), 0.77-0.60 (m, 16H), 0.60-0.38 (m, 16H) 0.55 (t, J= 7.3 Hz, 12H) ; APCI-FTMS (M⁺), calc. 1936.20319, obs. 1936.20494.

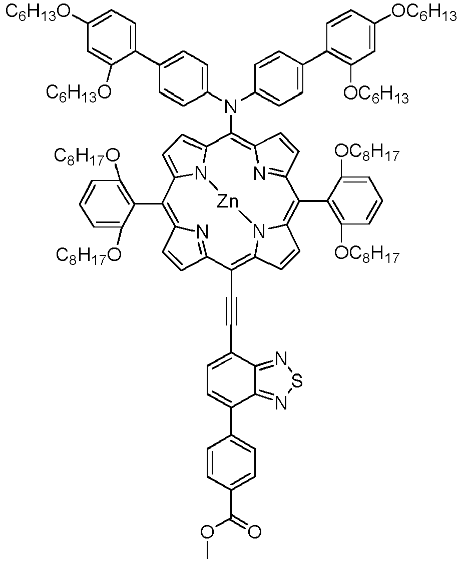

To a solution of Hag2N-ZnP(OC8)-TIPSA (222 mg, 0.1145 mmol) in THF (11.5 mL) was added TBAF (1M in THF, 286 µL, 0.286 mmol) and the resulting solution stirred for 30 minutes at room temperature. Water (50 mL) was added and the organics extracted with DCM (2 x 50 mL). The organics were dried (Na₂SO₄) filtered and evaporated. To the porphyrinic residue was added **Br-BTD-Ph-CO2Me** (80 mg, 0.229 mmol, 2 equiv), AsPh₃ (70.1 mg, 0.229 mmol, 2 equiv), Pd₂(dba)₃ (21 mg, 0.023 mmol, 20mol %), THF (11.5 mL) and Et₃N (2.3 mL). The solution was heated at reflux for 15 hours, prior to evaporation of the solvents and purification by column chromatography (silica, DCM/ hexane, 2:1) to afford the desired product (201 mg, 86 %) as a brown solid.

¹H NMR (400 MHz, CDCl₃) *δ* 9.95 (d, *J*= 4.4 Hz, 2H), 9.12 (d, *J*= 4.4 Hz, 2H), 8.90 (d, *J* = 4.4 Hz, 2H), 8.65 (d, *J* = 4.4 Hz, 2H), 8.26 (d, *J* = 8.2 Hz, 2H), 8.24 (d, *J* = 6.6 Hz, 1H), 8.17 (d, *J* = 8.2 Hz, 2H), 7.93 (d, *J* = 6.6 Hz, 1H), 7.66 (t, *J* = 8.0 Hz, 2H), 7.33 (s, 2H), 7.24 (d, *J* = 8.6 Hz, 4H), 7.17 (d, *J* = 8.6 Hz, 4H), 7.12 (d, *J* = 8.6 Hz, 2H), 6.98 (d, *J* = 8.5 Hz, 4H), 6.48 (s, 2H), 6.44 (d, *J*= 8.5 Hz, 2H), 3.99 (s, 3H), 3.92 (t, *J*= 6.8 Hz, 4H), 3.88 (t, *J*= 6.8 Hz, 4H), 3.84 (t, *J*= 6.4 Hz, 8H), 1.80-1.65 (m, 8H), 1.49-1.35 (m, 8H), 1.34-1.21 (m, 16H), 0.98-0.81 (m, 28H), 0.79-0.59 (m, 16H), 0.57-0.40 (m, 16H), 0.54 (t, J= 7.2 Hz, 12H); APCI-FTMS (M⁺), calc. 2048.09986, obs. 2048.10353.

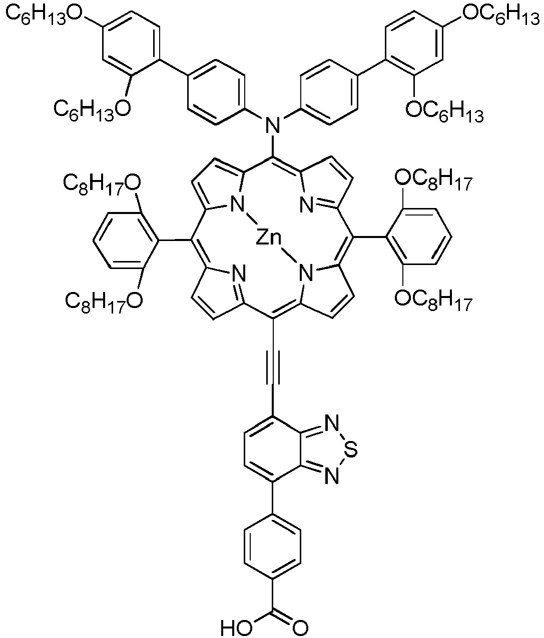

**Hag2N-ZnP(OC8)-BTD-PhCO2Me** (153 mg, 0.075 mmol) was dissolved in THF (30 mL), MeOH (20 mL) and a solution of NaOH (20 % w/w in water, 8 mL) was added. The solution was heated at 40°C for 2 hours upon which TLC (silica, DCM) indicated complete hydrolysis of the ester. The reaction mixture was diluted with Et₂O (100 mL) washed with water (100 mL), HCl (1M, 120 mL), water (100 mL), the organics dried (Na₂SO₄) and evaporated. The residue was loaded onto a short column (silica, DCM then 1:9 MeOH/DCM) to afford a brown solid which was further purified by recrystallization (Et₂O/MeOH) to afford the final product (115 mg, 75 %) as a brown solid.

¹H NMR (400 MHz, CDCl₃ + C₅D₅N) *δ* 9.94 (d, *J=* 4.4 Hz, 2H), 9.10 (d, *J=* 4.4 Hz, 2H), 8.88 (d, *J=* 4.4 Hz, 2H), 8.65 (d, *J=* 4.4 Hz, 2H), 8.34 (d, *J =* 8.2 Hz, 2H), 8.23 (d, *J* = 8.0 Hz, 1H), 8.17 (d, *J* = 8.2 Hz, 2H), 7.93 (d, *J=* 8.0 Hz, 1H), 7.64 (t, *J=* 8.4 Hz, 2H), 7.22 (d, *J=* 8.8 Hz, 4H), 7.15 (d, *J =* 8.8 Hz, 4H), 7.10 (d, *J* = 8.5 Hz, 2H), 6.96 (d, *J* = 8.5 Hz, 4H), 6.46 (s, 2H), 6.42 (d, *J =* 6.2Hz, 2H), 3.90 (t, *J =* 6.6 Hz, 4H), 3.88 (t, *J =* 6.6 Hz, 4H), 3.82 (t, *J=* 6.6 Hz, 8H), 1.78-1.62 (m, 8H), 1.47-1.33 (m, 8H), 1.32-1.17 (m, 16H), 0.99-0.79 (m, 32H), 0.78-0.58 (m, 16H), 0.52 (t, J= 7.2 Hz, 24H) ; APCI-FTMS (M⁺), calc. 2034.08476, obs. 2034.08453.

### Example 2 : Fabrication of Dye-Sensitized Solar Cells

The FTO glass substrates were immersed in 40 mM TiCl₄ aq. at 70°C for 30 min and washed with water and ethanol. The 8 µm thick mesoporous nano-TiO₂ films composed of 20 nm anatase TiO₂ particles were coated on the FTO glass plates by repetitive screen printing. After drying the nanocrystalline Ti02 layer at 125°C, a 4 µm thick second layer of 400 nm sized light scattering anatase particles (CCIC, HPW-400) was deposited by screen printing onto the transparent layer. The TiO₂ electrodes were gradually heated under an air flow at 325°C for 5 min, at 375°C for 5 min, at 450°C for 15 min and 500°C for 15 min. The TiO₂ electrodes were treated again by TiCl₄ and sintered at 500°C for 30 min. The TiO₂ electrode adsorbed with the dye compound (I-1) according to Example 1 and thermally platinized counter electrode were assembled into a sealed sandwich type cell with a gap of a hot-melt ionomer film (Surlyn 1702, 25 µm thickness, Du-Pont) as a spacer between the electrodes. A drop of electrolyte solution was placed on the drilled hole in the counter electrode of the assembled cell and was driven into the cell via vacuum backfilling, then the hole was sealed using additional Surlyn and a cover glass.

### Example 3 : Evaluation of Dye-Sensitized Solar Cells

The measurement of incident photon-to-current conversion efficiency (IPCE) was plotted as a function of excitation wavelength by using the incident light from a 300 W xenon lamp (ILC Technology, USA), which was focused through a Gemini-180 double monochromator (Jobin Yvon Ltd.). In order to reduce scattered light from the edge of the glass electrodes of the dye-adsorbed TiO₂ layer, a light shading mask was used onto the DSSCs, so active area of DSSCs was fixed to 0.159 cm². The irradiation source was a 450 W xenon light source (Osram XBO 450, USA), whose power of an AM 1.5 solar simulator was calibrated by using a reference Si photodiode equipped with an IR cutoff filter (KG-3, Schott) in order to reduce the mismatch in the region of 350-750 nm between the simulated light and AM 1.5 to less than 2 %. The measurement delay time of photo I-V characteristics of DSSCs was fixed to 40 ms.

**Table 1 : Efficiency (η) measurements for the DSSC according to Example 2**

| Dye | η under 9.4 % Sun | η under 50.9 % Sun | η under 64.1 % Sun | η under 98.8 % Sun |
|---|---|---|---|---|
| Example 1 | 13.35 % | 13.44 % | 13.59 % | 13.02 % |

## Claims

1. A compound of formula (I) :
D-B-A-ANC (I)
wherein D is an electron donor group, B is a chromophore group, A is an electron acceptor group, and ANC is an anchoring part comprising at least one anchoring group.

2. The compound according to Claim 1, wherein the chromophore group comprises at least one non-metal or metal complex chromophore, preferably selected from the group consisting of porphyrins, benzoporphyrins, phthalocyanines, naphthalocyanines and azulenocyanines, more preferably at least one porphyrin.

3. The compound according to Claim 2, wherein the porphyrin is selected from the following structures : 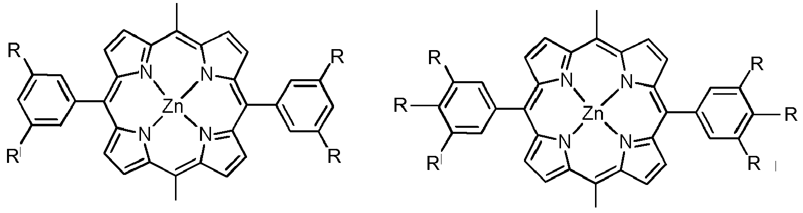 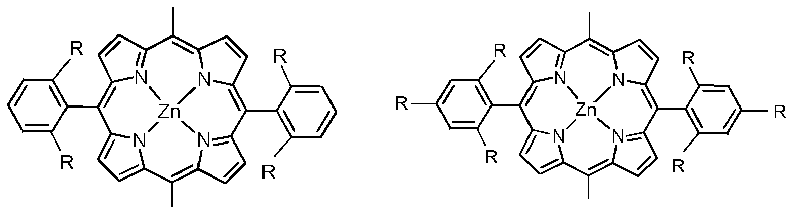 wherein R is independently selected from the group consisting of hydrogen, -CN, optionally branched alkyl, alkoxy and alkylthio groups and optionally substituted aryl, aryloxy and alkoxyaryl groups, particularly from optionally branched C1-C18 alkyl, alkoxy and alkylthio groups and C5-C12 aryl groups substituted by C8-C12 alkoxy groups, more particularly t-butyl, -OC₈H₁₇ or -OC₁₂H₂₅.

4. The compound according to any one of Claims 1 to 3, wherein the electron acceptor group A comprises at least one group selected from the following structures, preferably benzothiadiazole structure : 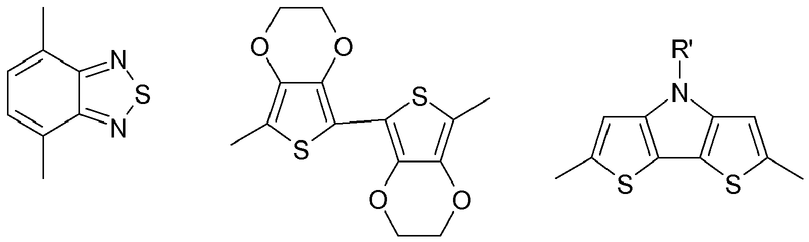 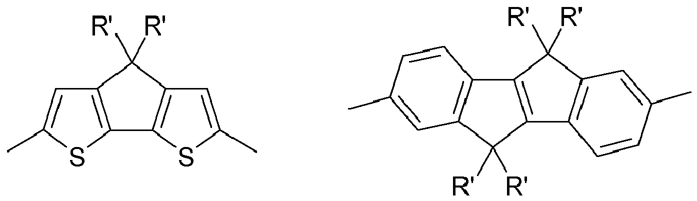 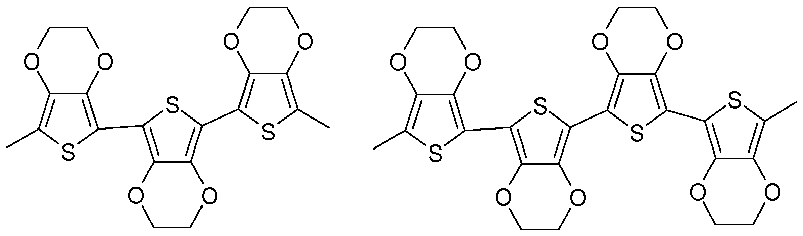 wherein R' are independently selected from the group consisting of optionally branched alkyl, alkoxy and alkylthio groups and optionally substituted aryl, aryloxy and alkoxyaryl groups, particularly from optionally branched C1-C18 alkyl, alkoxy and alkylthio groups and optionally substituted C5-C12 aryl groups, particularly from optionally branched C6-C12 alkyl groups and optionally substituted C6 aryl groups, more particularly from linear C6-C12 alkyl groups and phenyl group.

5. The compound according to any one of Claims 1 to 4, wherein the electron donor group D comprises optionally substituted amino group, preferably comprises at least one group selected from the following structures : 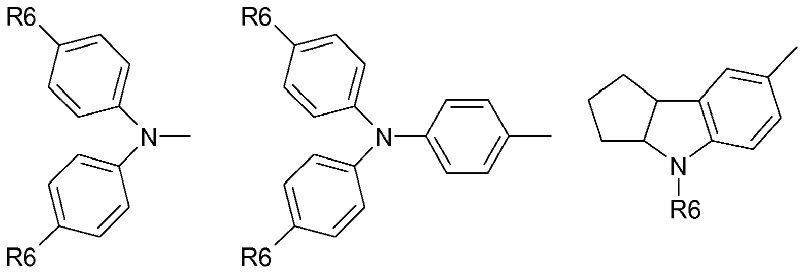 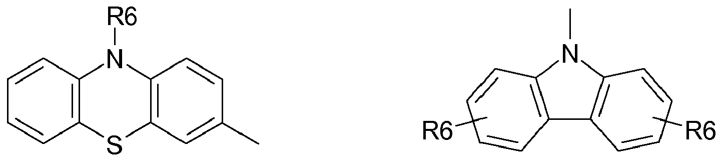 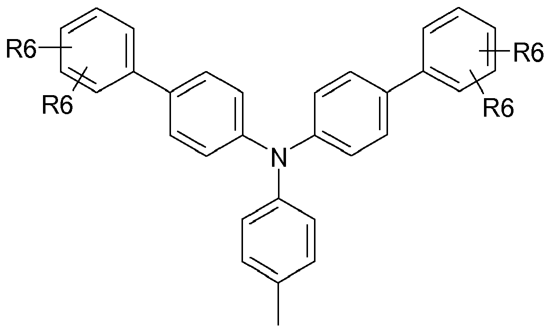 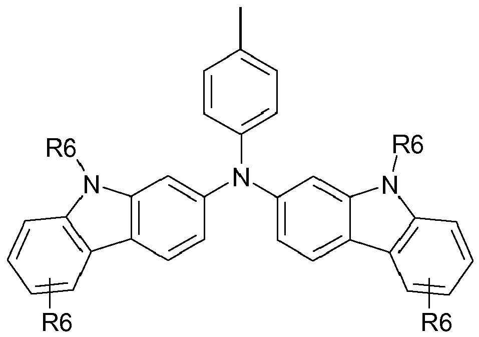 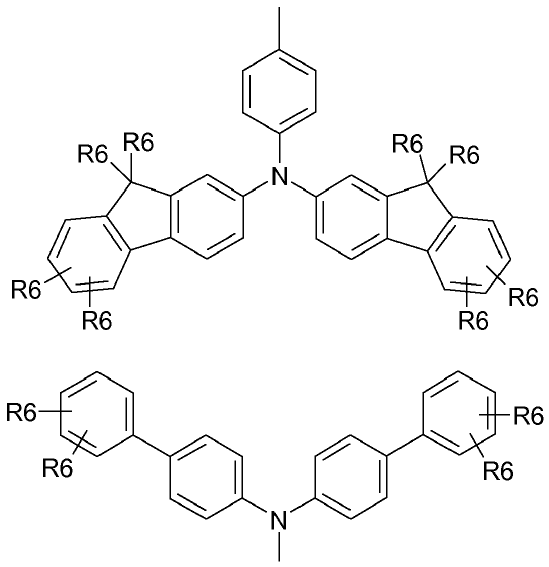 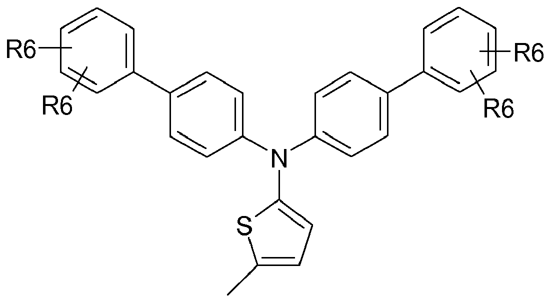 wherein R6 are independently selected from the group consisting of hydrogen, -CN, optionally branched C1-C18 alkyl, alkoxy and alkyothio groups and optionally substituted C6-C12 aryl, aryloxy and alkoxyaryl groups, preferably from optionally branched C1-C12 alkyl, alkoxy or alkylthio groups.

6. The compound according to any one of Claims 1 to 5, wherein the anchoring part comprises at least one aromatic group, preferably phenyl or naphthyl, and at least one anchoring group.

7. The compound according to any one of Claims 1 to 6, wherein the anchoring group is selected from the group consisting of -COOH, -PO₃H₂, -P(=O)(Ra)(OH) (wherein Ra is selected from the groups consisting of optionally branched C1-C18 alkyl groups and optionally substituted C5-C12 aryl groups, and halogenated derivatives thereof), -PO₄H₂, -PO₂HRb (wherein Rb is selected from alkyl groups or aryl groups), -SO₃H, -CONHOH, -NO₂, acetylacetonate, acrylic acid, preferably from H, CN, COOH and CF₃, malonic acid derivative, rhodanine-3-acetic acid, propionic acid, salicylic acid, formic anhydride, deprotonated forms of the aforementioned, salts of said deprotonated forms, and chelating groups with π-conducting character.

8. The compound according to any one of Claims 1 to 7, wherein D and B, and/or A and B are independently linked through an optional electron bridge group, the optional electron bridge group being independently selected from the group consisting of aromatic ring system, alkene group, polyene system, alkyne group, polyyne system or heteroatom-containing variants of such systems wherein one or more carbon atoms and/or one or more C=C double bonds are replaced by a heteroatom, particularly from alkene group, polyene system and phenyl moiety, more particularly from alkyne, -(CH=CH)n- where n is an integer from 1 to 10, phenyl groups and combinations thereof; most preferably from alkyne, -(CH=CH)n- where n is an integer from 1 to 3, phenyl groups and combinations thereof, especially phenyl.

9. The compound according to any one of Claims 1 to 8, wherein A and ANC are linked through an optional electron bridge group, the optional bridge group being independently selected from the group consisting of alkene group, polyene system, alkyne group, polyyne system or heteroatom-containing variants of such systems wherein one or more carbon atoms and/or one or more C=C double bonds are replaced by a heteroatom.

10. The compound according to any one of Claims 1 to 9, selected from the following structures : 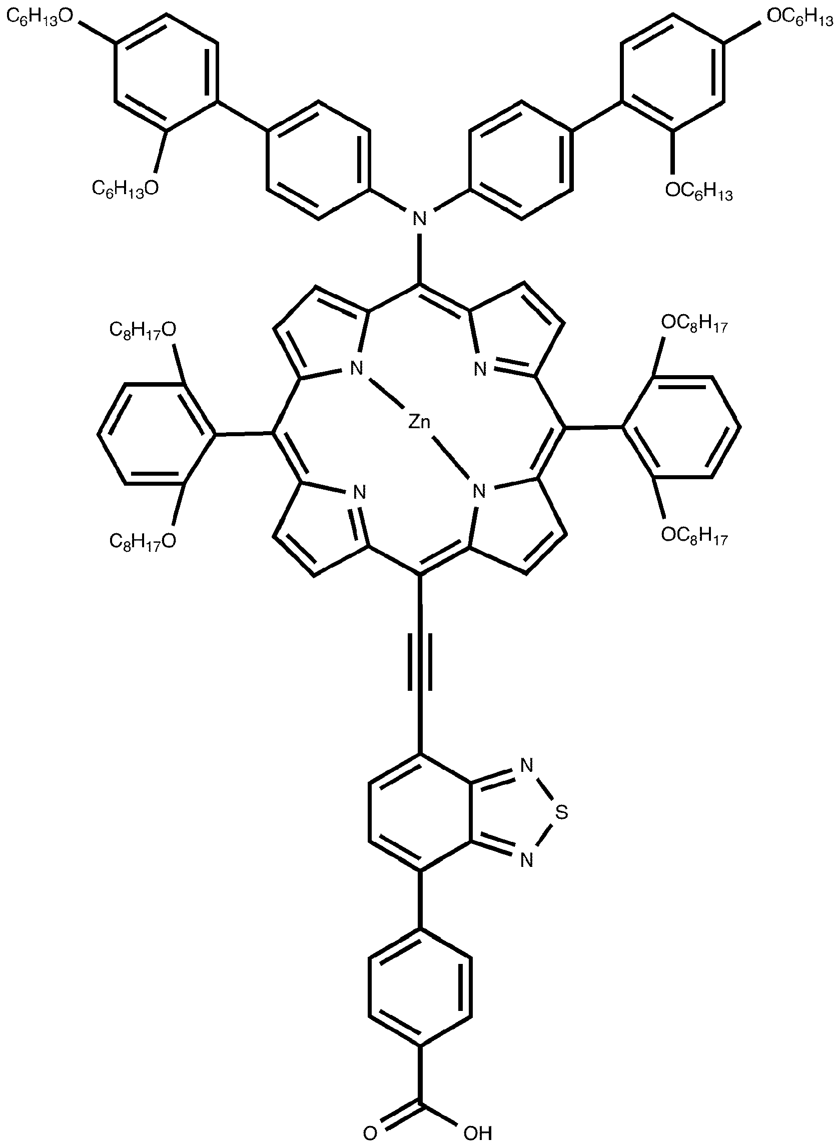 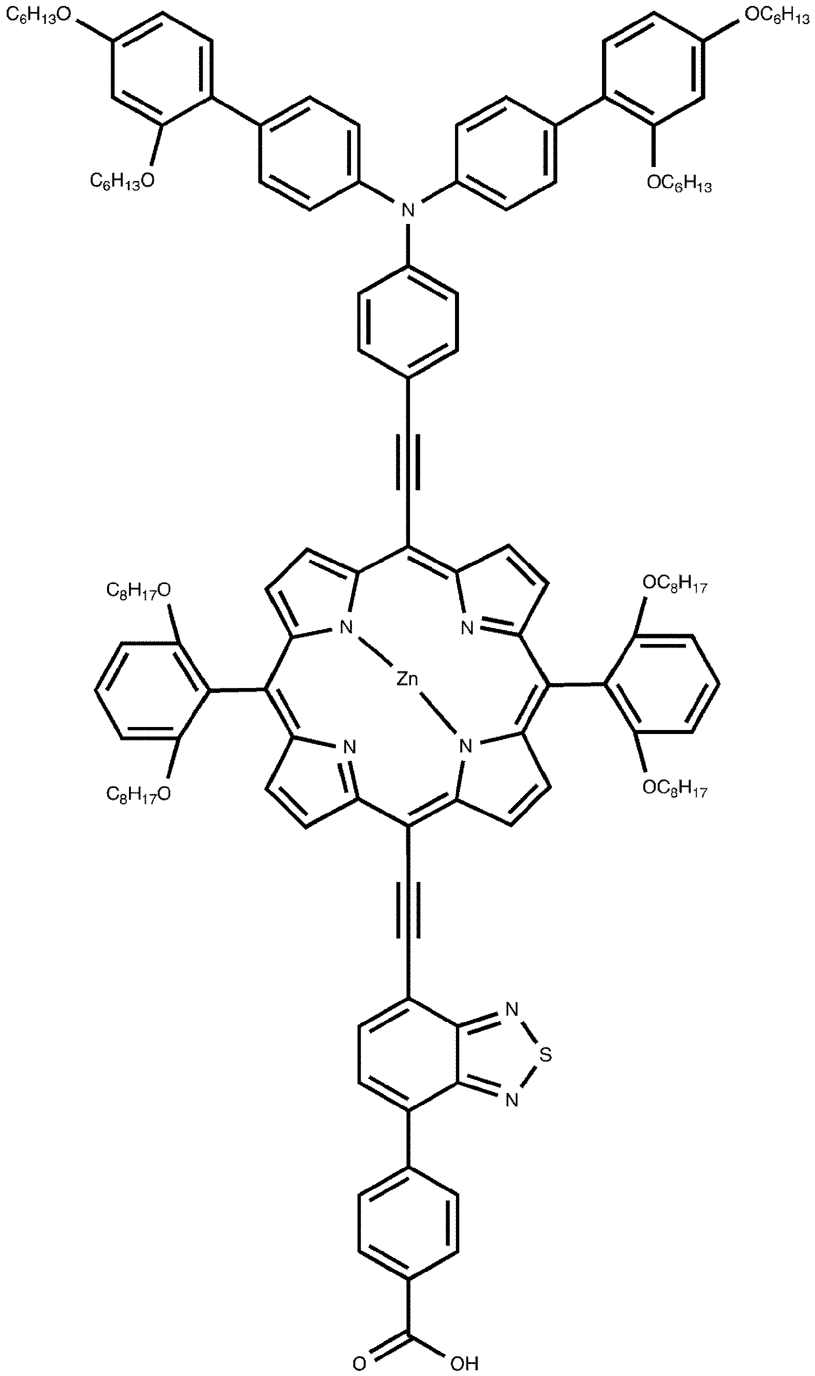 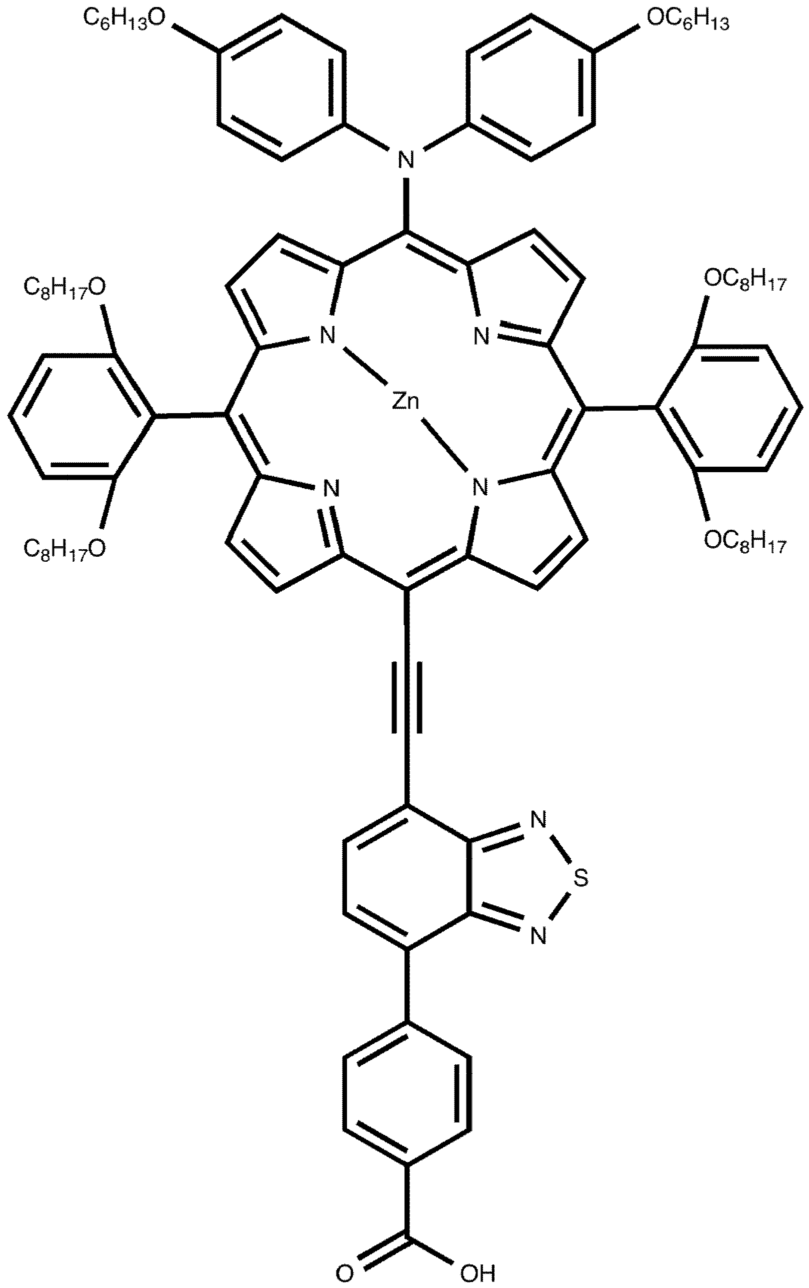 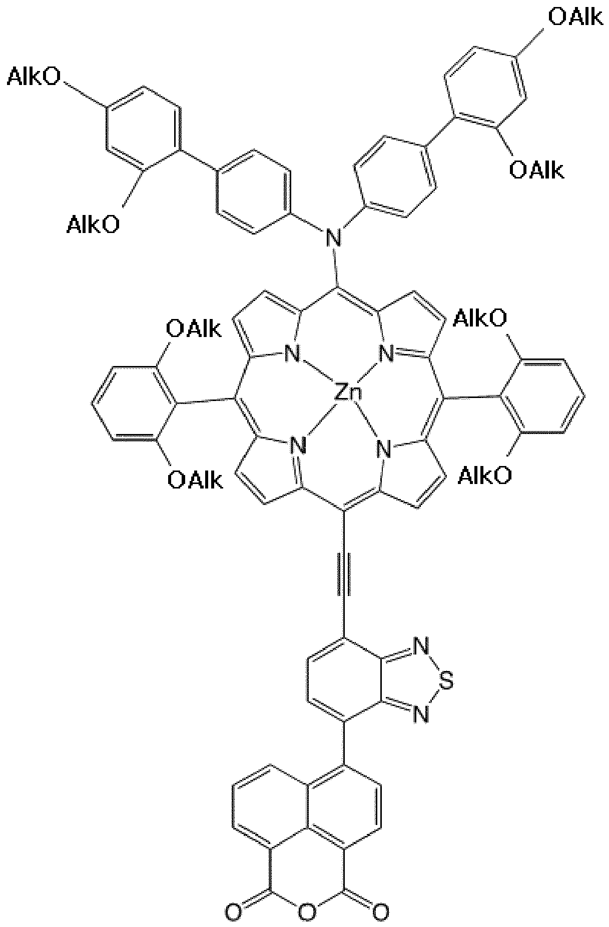 wherein Alk is alkyl group.

11. A salt, preferably an ammonium or alkali metal salt of the compound according to any one of Claims 1 to 10.

12. Use of the compound according to any one of Claims 1 to 10, or the salt according to Claim 11, in a photoelectric conversion device, in particular in a dye-sensitized solar cell.

13. A dye-sensitized solar cell comprising the compound according to any one of Claims 1 to 10 or the salt according to Claim 11.

14. The dye-sensitized solar cell according to Claim 13, wherein the compound is provided anchored to a semiconductor material, preferably the semiconductor material is selected from the group of titanium salts, tin salts and zinc salts, more preferably the semiconductor material is selected from the group of TiO₂, SnO₂, ZnO and TiOF₂.

15. The dye-sensitized solar cell according to Claim 13 or 14, further comprising a complex of a first row transition metal, preferably cobalt complex, as a redox mediator.
